# FASCICULE DE BREVET EUROPEEN

(11) **EP 2 326 284 B1**
(45) Date de publication et mention de la délivrance du brevet: **20.04.2016**
(21) Numéro de dépôt: 09749844.8
(22) Date de dépôt: 19.05.2009
(51) Int. Cl.: A61F 2/16

(54) **LENTILLE INTRAOCULAIRE**
INTRAOKULARLINSE
INTRAOCULAR LENS

(30) Priorité: 21.05.2008 FR 0802761
(43) Date de publication de la demande: 01.06.2011
(73) Titulaire: Medicontur Orvostechnikai Korlátolt Felelösségü Társaság, 2072 Zsámbék (HU)
(72) Inventeur: KONTUR, László, 80638 München (DE); TURKEVI-NAGY, Nándor, 2072 Zsámbék (HU)
(74) Mandataire: Laget, Jean-Loup
(86) Numéro de dépôt international: PCT/EP2009/056084
(87) Numéro de publication internationale: WO 2009/141354

(56) Documents cités:
- EP-A- 0 195 881
- EP-A- 0 246 216
- EP-A- 0 413 057
- FR-A- 2 814 671
- FR-A- 2 819 713
- FR-A- 2 858 544
- FR-A- 2 876 275
- US-A- 5 047 052

## Description

La présente invention a pour objet une lentille intraoculaire.

Une lentille intraoculaire comporte une partie optique, formant le système optique correcteur proprement dit, et une partie haptique, qui sert à la mise en place et à la fixation de la partie optique à l'intérieur de l'oeil dans la position convenable.

Une telle lentille est généralement réalisée dans un matériau souple permettant de la plier ou de la rouler, pour pouvoir l'insérer dans l'oeil par une incision de taille réduite, la lentille reprenant sa forme et ses dimensions normales lorsqu'elle est mise en place dans l'oeil.

Les lentilles intraoculaires doivent répondre à un certain nombre de contraintes mécaniques. En particulier, la partie haptique de la lentille doit être suffisamment souple pour bien tendre le sac capsulaire quelque soit la taille de l'oeil dans lequel la lentille est mise en place. La partie haptique de la lentille doit cependant être suffisamment rigide dans le plan optique pour permettre une correction efficace.

La forme de la lentille intraoculaire doit être adaptée pour limiter la migration de cellules, celles-ci entraînant une opacification de la capsule postérieure (PCO). Pour lutter contre l'opacification de la capsule postérieure, on réalise en général un bord carré sur toute la périphérie de la partie optique.

Pour cette raison, les lentilles intraoculaires sont généralement réalisées par fraisage puis tournage. Le fraisage permet de découper la lentille dans un bloc de matériau souple et de réaliser le bord carré sur la périphérie de la partie optique. Puis, le tournage permet de réaliser le bord carré au niveau des transitions entre la partie optique et la partie haptique. Cela peut entraîner des problèmes de décentrage lors du changement d'outils.

Le document D1 (FR-A-2 858 544 décrit une lentille intraoculaire monobloc souple destinée à être implantée dans le sac capsulaire après ablation du cristallin. Cette lentille comporte une partie optique et une partie haptique comprenant deux bras s'étendant radialement par rapport à la partie optique. Chaque bras comprend une partie courante et une partie de raccordement au bord de la partie optique ayant, selon la direction de l'axe optique, une épaisseur inférieure à celle de la partie courante pour former une ligne de flexion. La partie de raccordement de chaque bras présente une section droite réduite par rapport à celle de la partie courante pour définir une ligne de flexion ou de rotation du bras par rapport à la partie optique qui est sensiblement tangente à la périphérie de la partie optique. Toutefois, ce document est muet sur le problème du décentrage évoqués ci-dessus et sur les moyens d'y porter remède. De plus, en raison du fait qu'il préconise la constitution d'un bord carré par la différence d'épaisseur entre la partie optique et la partie haptique, son enseignement n'est pas applicable aux lentilles intraoculaires concernées par la présente invention dont la zone de raccordement interrompt le bord carré réalisé à la périphérie de la partie optique.

La présente invention a pour but de proposer une lentille intraoculaire qui réponde aux contraintes mécaniques ci-dessus décrites, qui évite les problèmes de décentrage lors de la fabrication et qui limite la migration de cellules.

A cet effet, l'invention a pour objet une lentille intraoculaire comportant une partie optique et une partie haptique, caractérisée en ce qu'elle comporte, au niveau de chaque transition entre ladite partie optique et ladite partie haptique, deux encoches, lesdites deux encoches étant disposées respectivement de part et d'autre de ladite transition, chaque encoche s'étendant le long du bord de ladite partie haptique et empiétant sur la périphérie de ladite partie optique.

Selon un premier mode de réalisation de l'invention, ladite partie haptique comprend deux ensembles haptiques disposés symétriquement par rapport au centre de ladite partie optique, chacun desdits deux ensembles haptiques comportant deux anses, chacune desdites deux anses comprenant une portion évidée entourée par un rebord.

De préférence, dans le premier mode de réalisation de l'invention, chacune desdites portions évidées est incluse dans un triangle orienté de manière que les deux axes entourant le sommet du triangle situé du côté de ladite partie optique coupent un diamètre de ladite partie optique, au dessus de l'axe optique de ladite partie optique.

Avantageusement, dans le premier mode de réalisation de l'invention, chacune desdites portions évidées empiète sur la périphérie de ladite partie optique.

De préférence, dans le premier mode de réalisation de l'invention, ledit ensemble d'encoches comporte quatre encoches, chaque encoche présentant une forme arrondie, s'étendant le long du bord d'une desdites anses, et empiétant sur la périphérie de ladite partie optique.

Selon un deuxième mode de réalisation de l'invention, ladite partie haptique comprend deux éléments diamétralement opposés, chacun desdits deux éléments comportant une portion de contact, destinée à venir en contact avec la paroi interne de l'oeil, et un bras de raccordement, raccordant ledit élément à la périphérie de ladite partie optique.

De préférence, dans le deuxième mode de réalisation de l'invention, ledit ensemble d'encoches comporte au moins deux encoches, chaque encoche présentant une forme ovoïde, s'étendant le long d'un bord d'un desdits bras de raccordement, et empiétant sur la périphérie de ladite partie optique.

L'invention sera mieux comprise, et d'autres buts, détails, caractéristiques et avantages de celle-ci apparaîtront plus clairement au cours de la description explicative détaillée qui va suivre, de plusieurs modes de réalisation de l'invention donnés à titre d'exemples purement illustratifs et non limitatifs, en référence aux dessins schématiques annexés.

Sur ces dessins :
- la figure 1 est une vue schématique simplifiée en élévation de la face postérieure d'une lentille intraoculaire selon un premier mode de réalisation de l'invention ;
- la figure 2 est une vue agrandie de la zone II de la figure 1 ;
- la figure 3 est une vue similaire à la figure 1 précisant la forme d'une anse de la partie haptique de la lentille intraoculaire ;
- la figure 4 est une vue similaire à la figure 1 montrant une lentille intraoculaire selon un deuxième mode de réalisation de l'invention ; et
- la figure 5 est une vue agrandie de la zone V de la figure 4.

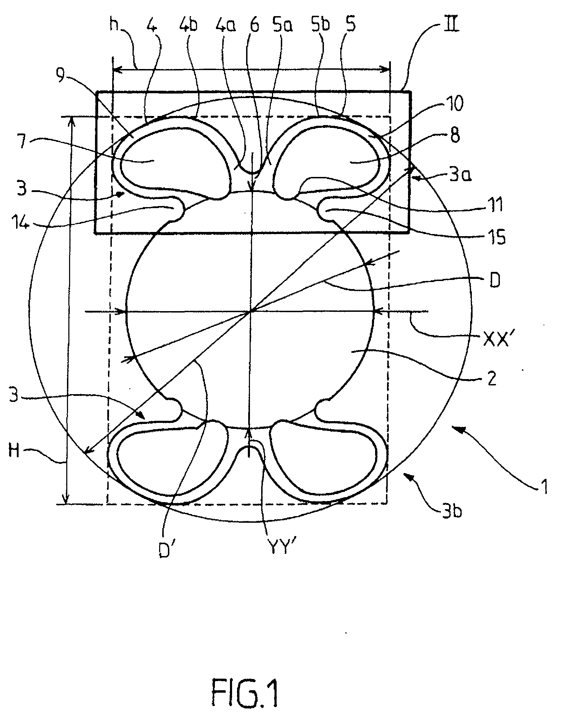

La figure 1 montre une lentille intraoculaire souple monobloc 1 selon un premier mode de réalisation de l'invention. La lentille 1 est destinée à être implantée dans le sac capsulaire d'un oeil (non représenté), d'une manière connue en soi.

La lentille intraoculaire 1 comporte une partie optique 2 et une partie haptique 3. Par exemple, la lentille intraoculaire 1 présente une hauteur H d'environ 9,74 mm, une largeur h d'environ 6,68 mm, et peut être incluse dans un cercle de diamètre D'environ égal à 10,7 mm. De manière générale, les dimensions de la lentille intraoculaire 1 sont adaptées aux dimensions d'un sac capsulaire.

La partie optique 2 est sensiblement circulaire et son diamètre D est par exemple environ égal à 6 mm.

La partie haptique 3 comprend deux ensembles haptiques 3a et 3b disposés symétriquement par rapport au centre de la partie optique 2, c'est-à-dire par rapport à l'intersection d'un diamètre X, X' de la partie optique 2 et d'un diamètre Y, Y' de la partie optique 2. Les deux ensembles haptiques 3a, 3b étant identiques, seul l'ensemble haptique 3a est décrit en détails ci-dessous.

L'ensemble haptique 3a comporte deux anses 4 et 5, chaque anse 4, 5 présentant une première extrémité 4a, 5a, respectivement, raccordée à la périphérie de la partie optique 2 et une deuxième extrémité 4b, 5b, respectivement, élastiquement déformable, destinée à venir au contact de la paroi interne de l'oeil lorsque la lentille 1 est mise en place dans l'oeil.

Les deux anses 4, 5 sont disposées sensiblement symétriquement par rapport au diamètre Y, Y'. L'ensemble haptique 3a comporte une bande de liaison 6 s'étendant entre les deux anses 4 et 5 le long de la périphérie de la partie optique 2.

Chaque anse 4, 5 comprend une portion évidée 7, 8, respectivement, qui est entourée par un rebord élastiquement déformable 9, 10, respectivement. Chaque rebord 9, 10 a par exemple une largeur d'environ 0,22 mm. L'une des portions évidée 7, 8, par exemple la portion évidée 8, comprend un détrompeur 11.

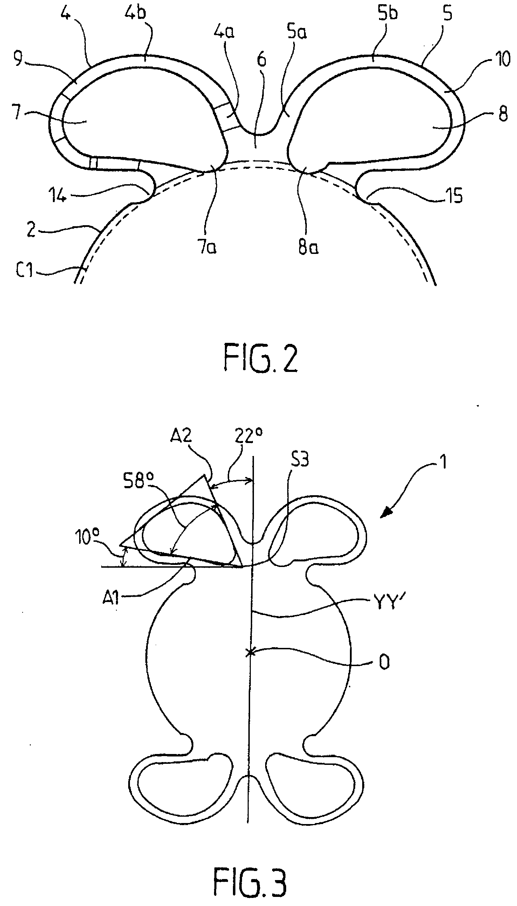

En se référant à la figure 3, on voit que chaque portion évidée 7, 8 est incluse dans un triangle orienté de manière que les deux axes A1 et A2 entourant le sommet S3 du triangle situé du côté de la partie optique 2 coupent le diamètre Y-Y' au dessus de l'axe optique O. Cette forme et cette orientation des portions évidées 7, 8 ont pour effet de réduire les forces de compression qui s'exercent sur les anses 4, 5 lorsque la lentille 1 est mise en place dans l'oeil. Il a été constaté expérimentalement que les forces de compression sont réduites d'environ un facteur deux par rapport aux forces s'exerçant sur des lentilles intraoculaires de l'art antérieur. De plus, cette forme et cette orientation des anses 4, 5 empêche ou du moins limite la présence de plis dans le sac capsulaire, ce qui évite la migration de cellules donc l'apparition de la PCO (opacification de la capsule postérieure).

Chaque portion évidée 7, 8 forme une encoche 7a, 8a, respectivement, qui empiète sur la périphérie de la partie optique 2, par exemple sur une épaisseur d'environ 0,1 mm. Cela a pour effet de diminuer le risque de passage de cellules, donc l'apparition de la PCO. Sur la figure 2, on a représenté en traits interrompus un arc de cercle C1 de même centre que la partie optique 2 pour mettre en évidence le retrait de matière sur la périphérie de la partie optique 2, c'est-à-dire les encoches 7a, 8a.

La lentille 1 comporte, au niveau de chaque anse 4, 5, une encoche 14, 15, respectivement, de forme arrondie, qui s'étend le long du bord de l'anse 4, 5 et empiète sur la périphérie de la partie optique 2, par exemple sur une épaisseur d'environ 0,1 mm (figure 2). Les encoches 14, 15 présentent trois fonctions.

En d'autres termes, la lentille 1 comporte, au niveau de chaque transition entre la partie optique 2 et la partie haptique 3, deux encoches 7a, 8a, 14, 15. Les deux encoches 7a, 8a, 14, 15 sont disposées respectivement de part et d'autre de la transition, chaque encoche 7a, 8a, 14, 15 s'étendant le long du bord de la partie haptique 3 et empiétant sur la périphérie de la partie optique 2.

Premièrement, les encoches 14, 15 servent de moyens de centrage pendant la fabrication de la lentille 1, ce qui évite les problèmes de décentrage lors du changement d'outils, et permet donc de diminuer le nombre de lentilles non utilisables. En effet, les encoches 7a, 8a, 14, 15 permettent de séparer la ligne de travail des outils, c'est-à-dire du tournage et du fraisage, en plusieurs secteurs. Chaque secteur peut ainsi être calibré séparément, ce qui facilite le centrage.

Deuxièmement, la présence d'encoches 14, 15 a pour conséquence la suppression de matière, ce qui a pour effet de limiter la migration de cellules, donc l'apparition de la PCO. En effet, il a été constaté expérimentalement que les cellules ont tendance à migrer le long de la périphérie de la partie optique 2, puis à passer sur la lentille 1 au niveau d'une transition entre la partie optique 2 et la partie haptique 3. Les encoches 7a, 8a, 14, 15 permettent d'avoir à coup sûr un bord carré au niveau de cette transition, et forment ainsi un réservoir retenant les cellules et empêchant leur migration sur la lentille 1.

Troisièmement, les encoches 14, 15 créent un point de flexion permettant aux anses 4, 5 de s'écarter vers l'extérieur au moment de la mise en place de la lentille 1, ce qui permet d'améliorer le maintien de la lentille 1 dans le sac capsulaire.

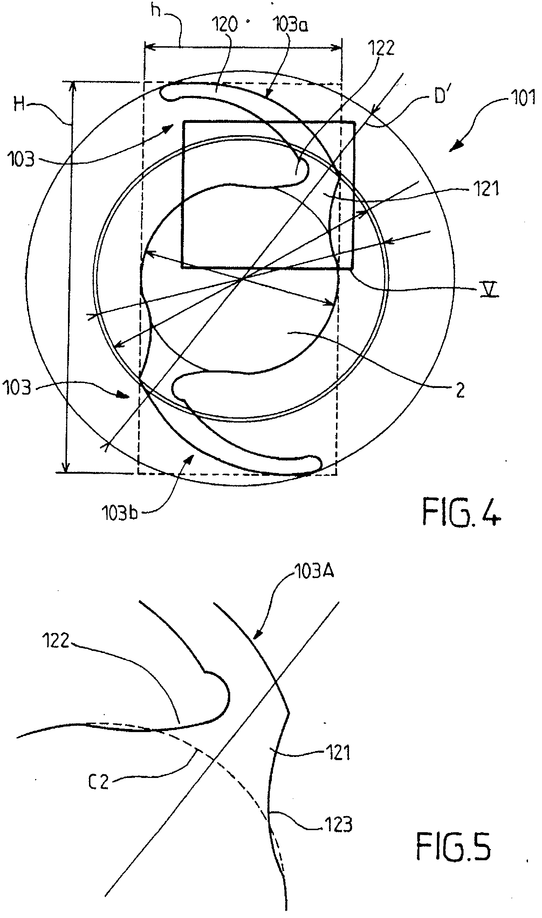

La figure 4 montre une lentille intraoculaire 101 selon un deuxième mode de réalisation. Les éléments similaires au premier mode de réalisation portent le même numéro de référence et ne seront pas décrits à nouveau.

Ici, la lentille intraoculaire 101 présente par exemple une hauteur H d'environ 12,30 mm, une largeur h d'environ 6 mm, et peut être incluse dans un cercle de diamètre D'environ égal à 13 mm.

La partie haptique 103 comprend deux éléments 103a et 103b diamétralement opposés. Les deux éléments 103a et 103b étant identiques, seul l'élément 103a est décrit ci-dessous. L'élément 103a comporte une portion de contact 120, destinée à venir en contact avec la paroi interne de l'oeil, et un bras de raccordement 121 raccordant l'élément 103a à la périphérie de la partie optique 2.

La lentille 101 comporte une encoche 122, de forme ovoïde, qui s'étend le long d'un bord du bras 121 et empiète sur la périphérie de la partie optique 2. La lentille 101 comporte une encoche 123, de forme ovoïde, qui s'étend le long de l'autre bord du bras 121 et empiète sur la périphérie de la partie optique 2.

La lentille 101 comporte donc, au niveau de chaque transition entre la partie optique 2 et la partie haptique 103, deux encoches 122, 123. Les deux encoches 122, 123 sont disposées respectivement de part et d'autre de la transition, chaque encoche 122, 123 s'étendant le long du bord de la partie haptique 103 et empiétant sur la périphérie de la partie optique 2.

Les encoches 122 et 123 présentent les mêmes fonctions que les encoches 14, 15 décrites précédemment. Sur la figure 5, on a représenté en traits interrompus un arc de cercle de même centre et de même rayon que la partie optique 2 pour mettre en évidence les retraits de matière sur la périphérie de la partie optique 2.

Bien que l'invention ait été décrite en relation avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Lentille intraoculaire (1, 101) comportant une partie optique (2) et une partie haptique (3, 103), la lentille intraoculaire (1, 101) comporte, au niveau de chaque transition entre ladite partie optique (2) et ladite partie haptique (3, 103), deux encoches (7a, 8a, 14, 15, 122, 123), lesdites deux encoches (7a, 8a, 14, 15, 122, 123) étant disposées respectivement de part et d'autre de ladite transition, chaque encoche (7a, 8a, 14, 15, 122, 123) s'étendant le long du bord de ladite partie haptique (3, 103), **caractérisée en ce que** chaque encoche (7a, 8a 14, 15, 122, 123) empiétant sur la périphérie de ladite partie optique (2).

2. Lentille intraoculaire selon la revendication 1, **caractérisée en ce que** ladite partie haptique (3) comprend deux ensembles haptiques (3a, 3b) disposés symétriquement par rapport au centre défini par l'intersection d'un diamètre X, X' et d'un diamètre Y, Y' de ladite partie optique 2, chacun desdits deux ensembles haptiques comportant deux anses (4, 5), chacune desdites deux anses comprenant une portion évidée (7, 8) entourée par un rebord (9, 10).

3. Lentille intraoculaire selon la revendication 2, **caractérisée en ce que** chacune desdites portions évidées (7, 8) est incluse dans un triangle orienté de manière que les deux axes (A1, A2) entourant le sommet (S3) du triangle situé du côté de ladite partie optique (2) coupent un diamètre (YY') de ladite partie optique passant par les bandes de liaison (6) entre les anses de chaque groupe haptique, au dessus de l'axe optique (O) de ladite partie optique.

4. Lentille intraoculaire selon la revendication 2 ou 3, **caractérisé en ce que** chacune desdites portions évidées (7, 8) empiète sur la périphérie de ladite partie optique (2).

5. Lentille intraoculaire selon l'une quelconque des revendications 2 à 4, **caractérisée en ce que** ledit ensemble d'encoches comporte quatre encoches (14, 15), chaque encoche présentant une forme arrondie, s'étendant le long du bord d'une desdites anses (4, 5), et empiétant sur la périphérie de ladite partie optique (2).

6. Lentille intraoculaire selon la revendication 1, **caractérisé en ce que** ladite partie haptique (103) comprend deux éléments (103a, 103b) diamétralement opposés, chacun desdits deux éléments comportant une portion de contact (120), destinée à venir en contact avec la paroi interne de l'oeil, et un bras de raccordement (121), raccordant ledit élément à la périphérie de ladite partie optique (2).

7. Lentille intraoculaire selon la revendication 6, **caractérisé en ce que** ledit ensemble d'encoches comporte au moins deux encoches (122, 123), chaque encoche présentant une forme ovoïde, s'étendant le long d'un bord d'un desdits bras de raccordement (121), et empiétant sur la périphérie de ladite partie optique (2).

## Patentansprüche

1. Intraokularlinse (1, 101), aufweisend einen optischen Teil (2) und einen haptischen Teil (3, 103), wobei die Intraokularlinse (1, 101) im Bereich jedes Übergangs zwischen dem optischen Teil (2) und dem haptischen Teil (3, 103) zwei Einkerbungen (7a, 8a, 14, 15, 122, 123) aufweist, wobei die zwei Einkerbungen (7a, 8a, 14, 15, 122, 123) jeweils auf der einen und der anderen Seite des Übergangs angeordnet sind, wobei sich jede Einkerbung (7a, 8a, 14, 15, 122, 123) entlang des Rands des haptischen Teils (3, 103) erstreckt, **dadurch gekennzeichnet, dass** jede Einkerbung (7a, 8a, 14, 15, 122, 123) über die Peripherie des optischen Teils (2) reicht.

2. Intraokularlinse nach Anspruch 1, **dadurch gekennzeichnet, dass** der haptische Teil (3) zwei haptische Einheiten (3a, 3b) umfasst, die in Bezug zum Zentrum des optischen Teils (2), das durch den Schnittpunkt eines Durchmessers X, X' und eines Durchmessers Y, Y' des optischen Teils 2 definiert ist, symmetrisch angeordnet sind, wobei jede der zwei haptischen Einheiten zwei Bügel (4, 5) aufweist, wobei jeder der zwei Bügel einen ausgesparten, von einer Einfassung (9, 10) umgebenen Abschnitt (7, 8) aufweist.

3. Intraokularlinse nach Anspruch 2, **dadurch gekennzeichnet, dass** jeder der ausgesparten Abschnitte (7, 8) in einem Dreieck eingeschlossen ist, das derart ausgerichtet ist, dass die zwei Achsen (A1, A2), welche die Spitze (S3) des Dreiecks, die sich auf der Seite des optischen Teils (2) befindet, einen Durchmesser (YY') des optischen Teils, der durch die Verbindungsstreifen (6) zwischen den Bügeln jeder haptischen Gruppe verläuft, oberhalb der optischen Achse (O) des optischen Teils schneiden.

4. Intraokularlinse nach Anspruch 2 oder 3, **dadurch gekennzeichnet, dass** jeder der ausgesparten Abschnitte (7, 8) über die Peripherie des optischen Teils (2) reicht.

5. Intraokularlinse nach einem der Ansprüche 2 bis 4, **dadurch gekennzeichnet, dass** die Einkerbungsgruppe vier Einkerbungen (14, 15) aufweist, wobei jede Einkerbung eine abgerundete Form aufweist, die sich entlang des Rands eines der Bügel (4, 5) erstreckt und über die Peripherie des optischen Teils (2) reicht.

6. Intraokularlinse nach Anspruch 1, **dadurch gekennzeichnet, dass** der haptische Teil (103) zwei diametral entgegengesetzte Elemente (103a, 103b) umfasst, wobei jedes der zwei Elemente einen Kontaktabschnitt (120) aufweist, der bestimmt ist, mit der Innenwand des Auges in Kontakt zu kommen, und einen Verbindungsarm (121), der das Element mit der Peripherie des optischen Teils (2) verbindet.

7. Intraokularlinse nach Anspruch 6, **dadurch gekennzeichnet, dass** die Einkerbungsgruppe mindestens zwei Einkerbungen (122, 123) aufweist, wobei jede Einkerbung eine eiförmige Form aufweist, die sich entlang eines Rands eines der Verbindungsarme (121) erstreckt und über die Peripherie des optischen Teils (2) reicht.

## Claims

1. An intraocular lens (1, 101) including an optical part (2) and a haptic part (3, 103), the intraocular lens (1, 101) having, at each transition between said optical part (2) and said haptic part (3, 103), two notches (7a, 8a, 14, 15, 122, 123), said two notches (7a, 8a, 14, 15, 122, 123) being respectively positioned on either side of said transition, each notch (7a, 8a, 14, 15, 122, 123) extending along the edge of said haptic part (3, 103), **characterized in that** each notch (7a, 8a, 14, 15, 122, 123) encroaches on the periphery of said optical part (2).

2. The intraocular lens according to claim 1, **characterized in that** said haptic part (3) comprises two haptic assemblies (3a, 3b) arranged symmetrically relative to the center defined by the intersection of a diameter X-X' and a diameter Y-Y' of said optical part 2, each of said two haptic assemblies including two loops (4, 5), each of said two loops comprising a hollow portion (7, 8) surrounded by a rim (9, 10).

3. The intraocular lens according to claim 2, **characterized in that** each of said hollow portions (7, 8) is included in a triangle oriented such that the two axes (A1, A2) surrounding the apex (S3) of the triangle situated on the side of said optical part (2) intersect a diameter (YY') of said optical part passing through the connecting bands (6) between the loops of each haptic group, above the optical axis (O) of said optical part.

4. The intraocular lens according to claim 2 or 3, **characterized in that** each of said hollow portions (7, 8) encroaches on the periphery of said optical part (2).

5. The intraocular lens according to any one of claims 2 to 4, **characterized in that** said set of notches includes four notches (14, 15), each notch having a rounded shape, extending along the edge of one of the loops (4, 5), and encroaching on the periphery of said optical part (2).

6. The intraocular lens according to claim 1, **characterized in that** said haptic part (103) comprises two diametrically opposite elements (103a, 103b), each of said two elements including a contact portion (120), designed to come into contact with the inner wall of the eye, and a connecting arm (121), connecting said element to the periphery of said optical part (2).

7. The intraocular lens according to claim 6, **characterized in that** said set of notches includes at least two notches (122, 123), each notch having an ovoid shape, extending along an edge of one of said connecting arms (121), and encroaching on the periphery of said optical part (2).
